# EUROPEAN PATENT APPLICATION

(11) **EP 4 332 632 A1**
(43) Date of publication of application: **06.03.2024**
(21) Application number: 22794931.0
(22) Date of filing: 27.04.2022
(51) Int. Cl.: G01S 17/89

(54) **THREE-DIMENSIONAL ULTRASONIC IMAGING METHOD AND SYSTEM BASED ON LASER RADAR**

(30) Priority: 28.04.2021 CN 202110466195
(71) Applicant: Telefield Medical Imaging Limited, Shatin, N.T., Hong Kong (HK)
(72) Inventor: ZHENG, Yongping, Hong Kong (CN)
(74) Representative: ZHAOffice SPRL
(86) International application number: PCT/CN2022/089536
(87) International publication number: WO 2022/228461

(57) **Abstract**

A three-dimensional ultrasonic imaging method and system based on a Lidar. The system comprises: an ultrasonic probe (10), used for performing ultrasonic scanning on a region of interest of a target object; a two-dimensional ultrasonic imaging device (15), used for generating two-dimensional ultrasound images of the region of interest of the target object on the basis of ultrasonic scanning; a three-dimensional space information acquisition device (12), used for acquiring three-dimensional space information of the ultrasonic probe (10) by means of the LiDAR (13); a three-dimensional reconstruction module (16), used for reconstructing a three-dimensional ultrasound image on the basis of the three-dimensional space information of the ultrasonic probe (10) and the two-dimensional ultrasound image; and a user terminal (20), used for displaying the three-dimensional ultrasound image. The system reconstructs the three-dimensional ultrasound image in a flexible, low-cost and small-size manner, thereby improving accessibility of the three-dimensional ultrasonic technology, and expanding application scenarios in the field of three-dimensional sensing.

## Description

### TECHNICAL FIELD

The present application relates to the field of three-dimensional ultrasonic imaging, more specifically, to a three-dimensional ultrasonic imaging method and system based on a LiDAR.

### BACKGROUND

Free-hand three-dimensional imaging refers to the scanning of the target object by manually moving the ultrasound probe, and the position and orientation information of the probe are captured using three-dimensional spatial sensing technology. Commonly used three-dimensional spatial sensing technologies include spatial reference objects or signals and their corresponding detectors. For example, an electromagnetic emitter can be used to emit electromagnetic waves as reference signals, and detectors can determine the position and direction changes of the probe based on the variations in the electromagnetic field strength. Another example is using one or more visual markers placed on the surface of the probe as reference objects and using one or more cameras surrounding the ultrasound probe to detect the position and orientation of the probe. Furthermore, there are also patents proposing three-dimensional ultrasonic imaging techniques based on three-dimensional tracking cameras. This method utilizes Time-of-Flight (ToF) cameras or binocular stereo cameras and can be applied in any scenario without specific reference objects.

The above three-dimensional sensing technologies each have their own advantages and limitations. For example, electromagnetic sensing technology can be interfered by surrounding metal objects. Camera-based sensing systems are typically large in size and costly. However, miniaturization and portability of ultrasonic imaging systems are the future development direction, and optimizing the large space positioning system is an important technical issue for promoting the development of three-dimensional ultrasound industry.

LiDAR, is an active sensing technology that uses infrared lasers as the emission source to precisely and rapidly acquire three-dimensional space information of targets. It finds applications in military, aerospace, as well as civilian fields such as unmanned driving, robot vision navigation, and remote sensing mapping. The working principle of LiDAR involves emitting infrared laser onto the surface of objects and then receiving the reflected light signals, comparing them with the initial laser to calculate the distance. In terms of three-dimensional imaging applications, the essence of this technology lies in measuring the flight time of light pulses or modulated signals between the radar and the target, to obtain distance information (Z-axis), while simultaneously acquiring azimuth (X-axis) and elevation (Y-axis) information within the plane perpendicular to the direction of the beam through scanning or correspondingly measuring multiple points. Specifically, to achieve three-dimensional imaging, each pixel within the three-dimensional space is subjected to LiDAR ranging. Based on single-point ranging, the azimuth information of each ranging point is measured synchronously, such as azimuth-elevation-distance or distance-velocity-intensity, and the data is displayed in the form of an image. However, so far, there is still a lack of a sensing system that applies LiDAR to free-hand three-dimensional ultrasonic imaging.

### SUMMARY

### Technical problems

The technical problem to be addressed by this application is to provide a three-dimensional ultrasonic imaging method and system based on a LiDAR that has strong anti-interference capability, low cost, small size, high accessibility, and can be used in any environment without the need for specific reference objects.

### Solutions to the problem

### Technical Solutions

To solve the above technical problems, the technical solution adopted by the application is to provide a three-dimensional ultrasonic imaging system based on a LiDAR, comprises:
an ultrasonic probe, used for performing ultrasonic scanning on a region of interest of a target object;
a two-dimensional ultrasonic imaging device, used for generating a series of two-dimensional ultrasound images of the region of interest of the target object on the basis of ultrasonic scanning;
a three-dimensional space information acquisition device, used for acquiring three-dimensional space information of the ultrasonic probe by means of the LiDAR;
a three-dimensional reconstruction module, used for reconstructing a three-dimensional ultrasound image on the basis of the three-dimensional space information of the ultrasonic probe and the series of two-dimensional ultrasound images.

In the three-dimensional ultrasonic imaging system based on a LiDAR of the present application, the three-dimensional space information acquisition device comprises: a LiDAR and a processing module; the LiDAR is fixedly connected to the ultrasonic probe, and moves synchronously with the ultrasonic probe; the LiDAR is used to acquire environmental depth data and generate an initial three-dimensional space information based on the environmental depth data; the processing module is used to convert the initial three-dimensional space information into three-dimensional space information of the ultrasound probe.

In the three-dimensional ultrasonic imaging system based on a LiDAR of the present application, when the ultrasound probe is displaced, the three-dimensional space information acquisition device converts the environmental depth data in real-time through the processing module to obtain a series of three-dimensional space information of the ultrasound probe.

In the three-dimensional ultrasonic imaging system based on a LiDAR of the present application, when the ultrasound probe is displaced, the three-dimensional space information acquisition device converts the environmental depth data in real-time through the processing module using simultaneous localization and mapping technology to obtain a series of three-dimensional space information of the ultrasound probe.

In the three-dimensional ultrasonic imaging system based on a LiDAR of the present application, the three-dimensional space information acquisition device comprises multiple LiDARs; the relative positions of the multiple LiDARs with respect to the ultrasound probe are different, or the orientations of the multiple LiDARs are different; the multiple LiDARs are used to acquire multiple sets of environmental depth data and generate multiple sets of initial three-dimensional space information based on the multiple sets of environmental depth data; the processing module is used to transform the multiple sets of initial three-dimensional space information and generate the three-dimensional space information of the ultrasound probe.

In the three-dimensional ultrasonic imaging system based on a LiDAR of the present application, the three-dimensional space information acquisition device comprises a LiDAR and a processing module; the LiDAR is separated from the ultrasound probe; the LiDAR is arranged in a position where at least one marker is within the visible range of the LiDAR, and is used to acquire a contour information of the marker and generate an initial three-dimensional space information of the ultrasound probe based on the contour information of the marker; the marker comprises at least a portion of the ultrasound probe and/or at least one visual marker set on the ultrasound probe; the processing module is used to convert the initial three-dimensional space information into three-dimensional space information of the ultrasound probe.

In the three-dimensional ultrasonic imaging system based on a LiDAR of the present application, the three-dimensional space information acquisition device comprises multiple LiDARs; the multiple LiDARs are positioned at different locations or facing different directions in space, and are used to capture multiple sets of contour information of the ultrasound probe or a portion thereof; based on the multiple sets of the contour information of the ultrasound probe or a portion thereof, multiple sets of initial three-dimensional space information of the ultrasound probe are generated; the processing module is used for converting the multiple sets of initial three-dimensional space information into the three-dimensional space information of the ultrasound probe.

In the three-dimensional ultrasonic imaging system based on a LiDAR of the present application, the three-dimensional space information acquisition device further comprises at least one motion sensor and/or at least one camera.

In the three-dimensional ultrasonic imaging system based on a LiDAR of the present application, the three-dimensional space information acquisition device is at least a part of a terminal device integrated with the LiDAR.

In the three-dimensional ultrasonic imaging system based on a LiDAR of the present application, the three-dimensional space information acquisition device also comprises a correction module; the correction module is used to correct the position of the initial three-dimensional space information and the two-dimensional ultrasound image in the three-dimensional space based on changes in the acquired initial three-dimensional space information and content of the series of two-dimensional ultrasound images.

In the three-dimensional ultrasonic imaging system based on a LiDAR of the present application, the three-dimensional ultrasonic imaging system further comprises an installation module that securely connects the three-dimensional space information acquisition device and the ultrasound probe; the installation module comprises a handle that can be gripped by an operator.

In the three-dimensional ultrasonic imaging system based on a LiDAR of the present application, the three-dimensional ultrasonic imaging system further comprises a data integration and communication device; the data integration and communication device is used to integrate the series of two-dimensional ultrasound images obtained from the two-dimensional ultrasonic imaging device and the three-dimensional space information obtained from the three-dimensional space information acquisition device, and transmit them to the three-dimensional reconstruction module through wired or wireless mode.

In the three-dimensional ultrasonic imaging system based on a LiDAR of the present application, the three-dimensional ultrasonic imaging system further comprises a cloud computing module; the cloud computing module is used to implement all or part of functions of the three-dimensional reconstruction module.

In the three-dimensional ultrasonic imaging system based on a LiDAR of the present application, the three-dimensional ultrasonic imaging system further comprises a user terminal; the user terminal is used to display the three-dimensional ultrasound image.

Another technical solution adopted by the present application to solve the above technical problems is to provide a three-dimensional ultrasonic imaging method based on a LiDAR, comprises the following steps:
step S 1. using an ultrasonic probe to perform ultrasonic scanning on a region of interest of a target object;
step S2. generating a series of two-dimensional ultrasound images of the region of interest of the target object on the basis of ultrasonic scanning;
step S3. acquiring three-dimensional space information of the ultrasonic probe by a three-dimensional space information acquisition device based on the LiDAR;
step S4. reconstructing a three-dimensional ultrasound image on the basis of the three-dimensional space information of the ultrasonic probe and the two-dimensional ultrasound image.

In the three-dimensional ultrasonic imaging method based on a LiDAR of the present application, the LiDAR is fixedly connected to the ultrasonic probe, and moves synchronously with the ultrasonic probe, the step S3 comprises:
step S31. acquiring environmental depth data by the LiDAR of the three-dimensional space information acquisition device;
step S32. generating an initial three-dimensional space information based on the environmental depth data;
step S33. converting the initial three-dimensional space information into three-dimensional space information of the ultrasound probe.

In the three-dimensional ultrasonic imaging method based on a LiDAR of the present application, the LiDAR is separated from the ultrasound probe; the LiDAR is arranged in a position where at least one marker is within the visible range of the LiDAR; the marker comprises at least a portion of the ultrasound probe and/or at least one visual marker set on the ultrasound probe; the step S3 comprises:
step S31. acquiring a contour information of the marker by the LiDAR of the three-dimensional space information acquisition device;
step S32. generating an initial three-dimensional space information of the ultrasound probe based on the contour information of the marker;
step S33. converting the initial three-dimensional space information of the ultrasonic probe into the three-dimensional space information of the ultrasound probe.

In the three-dimensional ultrasonic imaging method based on a LiDAR of the present application, in the step S3, when the ultrasound probe is displaced, converting an environmental depth data in real-time to obtain a series of three-dimensional space information of the ultrasound probe.

In the three-dimensional ultrasonic imaging method based on a LiDAR of the present application, in the step S3, when the ultrasound probe is displaced, converting an environmental depth data in real-time using simultaneous localization and mapping technology to obtain a series of three-dimensional space information of the ultrasound probe.

In the three-dimensional ultrasonic imaging method based on a LiDAR of the present application, the step S4 comprises the following steps:
S41. transferring the three-dimensional space information of the ultrasonic probe and the series of two-dimensional ultrasound images to a cloud server;
S42. based on the three-dimensional space information of the ultrasonic probe and the two-dimensional ultrasound image, reconstructing the three-dimensional ultrasound image at the cloud server;
S43. transferring the three-dimensional ultrasound image to a user terminal for display.

### Beneficial effects of the application

### Beneficial effects

The implementation of the three-dimensional ultrasonic imaging method and system based on a LiDAR in this application can achieve at least the following beneficial effects: the three-dimensional ultrasonic imaging method based on a LiDAR in this application obtain three-dimensional space information using LiDAR to reconstruct the three-dimensional ultrasound image in a flexible, low-cost and small-size manner, effectively avoiding interference, without the need for specific reference objects. Furthermore, this application can also integrate existing two-dimensional ultrasonic imaging device with intelligent terminals equipped with integrated LiDAR to build a three-dimensional ultrasonic imaging system at the device application level, promoting miniaturization and portability of ultrasound equipment, thereby improving accessibility of the three-dimensional ultrasonic technology, and expanding application scenarios in the field of three-dimensional sensing.

### Brief description of the accompanying drawings

### DESCRIPTION OF THE DRAWINGS

For a clearer explanation of the technical solutions in the embodiments or prior art described, a brief introduction to the accompanying drawings required in the embodiments or description of the prior art will be provided below. It is evident that the accompanying drawings described below are only embodiments of the application, and ordinary skilled person in the field can obtain additional accompanying drawings based on the provided drawings without exercising inventive labor.
Figure 1 is a block diagram for the three-dimensional ultrasonic imaging system based on a LiDAR provided by the first embodiment of the present application;
Figure 2 is a three-dimensional structure diagram for the three-dimensional ultrasonic imaging system based on a LiDAR provided by the first embodiment of the present application;
Figure 3 is a three-dimensional structure diagram for the three-dimensional ultrasonic imaging system based on a LiDAR provided by the second embodiment of the present application;
Figure 4 is a three-dimensional structure diagram for the three-dimensional ultrasonic imaging system based on a LiDAR provided by the third embodiment of the present application;
Figure 5 is a three-dimensional structure diagram for the three-dimensional ultrasonic imaging system based on a LiDAR provided by the fourth embodiment of the present application;
Figure 6 is a three-dimensional structure diagram for the three-dimensional ultrasonic imaging system based on a LiDAR provided by the fifth embodiment of the present application;
Figure 7 is a three-dimensional structure diagram for the three-dimensional ultrasonic imaging system based on a LiDAR provided by the sixth embodiment of the present application;
Figure 8 is a three-dimensional structure diagram for the three-dimensional ultrasonic imaging system based on a LiDAR provided by the seventh embodiment of the present application;
Figure 9 is a three-dimensional structure diagram for the three-dimensional ultrasonic imaging system based on a LiDAR provided by the eighth embodiment of the present application;
Figure 10 is a three-dimensional structure diagram for the three-dimensional ultrasonic imaging system based on a LiDAR provided by the ninth embodiment of the present application;
Figure 11 is a three-dimensional structure diagram for the three-dimensional ultrasonic imaging system based on a LiDAR provided by the tenth embodiment of the present application;
Figure 12 is a flowchart of the steps for the three-dimensional ultrasonic imaging method based on a LiDAR provided by the tenth embodiment of the present application.

### Embodiments of the application

### DETAILED DESCRIPTION OF THE PREFERRED EMBODIMENT

As is well known, traditional three-dimensional positioning devices typically require special reference objects to obtain accurate three-dimensional space information. Even tracking cameras that don't require special reference objects, such as ToF cameras or stereo cameras, have drawbacks such as being bulky, expensive, sensitive to lighting conditions, and high requirements on the surface characteristics (i.e., marker) of the target object.

LiDAR, on the other hand, utilizes infrared laser to detect the surrounding environment and obtain three-dimensional information about the positions and orientations of the markers in the three-dimensional space. In addition to laser ranging, LiDAR can further refine the three-dimensional space information using built-in motion sensors such as accelerometers, gyroscopes, magnetometers, or inertial measurement units, along with RGB cameras. Therefore, LiDAR can accurately retrieve the three-dimensional space information of the markers without the need for special reference objects. Commercialized products that incorporate LiDAR scanning cameras include Velodyne's VLP-16, Intel's Realsense L515, and smartphones such as Apple's iPhone 12 Pro, iPad Pro, Samsung's Galaxy S20+, S20 Ultra, and others.

The concept of this application is to combine a two-dimensional ultrasonic imaging device with a LiDAR, using the three-dimensional space information of the ultrasonic probe provided by the LiDAR and the series of two-dimensional ultrasonic images information provided by the two-dimensional ultrasonic imaging device for three-dimensional image reconstruction. Further, the application proposes using the ultrasonic probe itself or a part of the probe as a marker, or setting the LiDAR on the ultrasonic probe to use objects within the visual range of the LiDAR as visual markers, thereby obtaining the motion trajectory and pose of the ultrasonic probe. Furthermore, by comparing the changes in three-dimensional space information measured by the LiDAR and the changes in two-dimensional image information of the target object collected by the ultrasonic probe, the errors caused by sudden changes in both can be corrected. Moreover, by setting multiple LiDAR sensors, cameras, and/or motion sensors, the accuracy of the three-dimensional space information can be improved, thereby improving the quality of the three-dimensional ultrasonic image. Finally, integrating the two-dimensional ultrasonic imaging device into a terminal configured with a LiDAR and building a three-dimensional ultrasonic imaging system at the equipment application level.

In general, the present application describes two technical approaches. One is to mount a LiDAR on an ultrasonic probe and use objects within the visual range of the LiDAR as visual markers. The other approach is to position the LiDAR in such a way that at least one marker is within its visual range, and the ultrasonic probe itself or a part of the probe serves as the marker. The following sections provide detailed explanations of the solutions implemented in embodiments one to seven based on these two technical approaches.

For a better understanding of the application, a more comprehensive description will be given with reference to the accompanying drawings. The drawings depict typical embodiments of the application. However, it should be noted that the application can be implemented in various forms and is not limited to the embodiments described herein. Instead, these embodiments are provided to enhance the thoroughness and comprehensiveness of the disclosure of the application.

Unless otherwise defined, all technical and scientific terms used in this document have the same meaning as understood by those skilled in the art of the application. The terms used in the description of the application are intended for the purpose of describing specific embodiments and are not intended to limit the application.

### The first embodiment

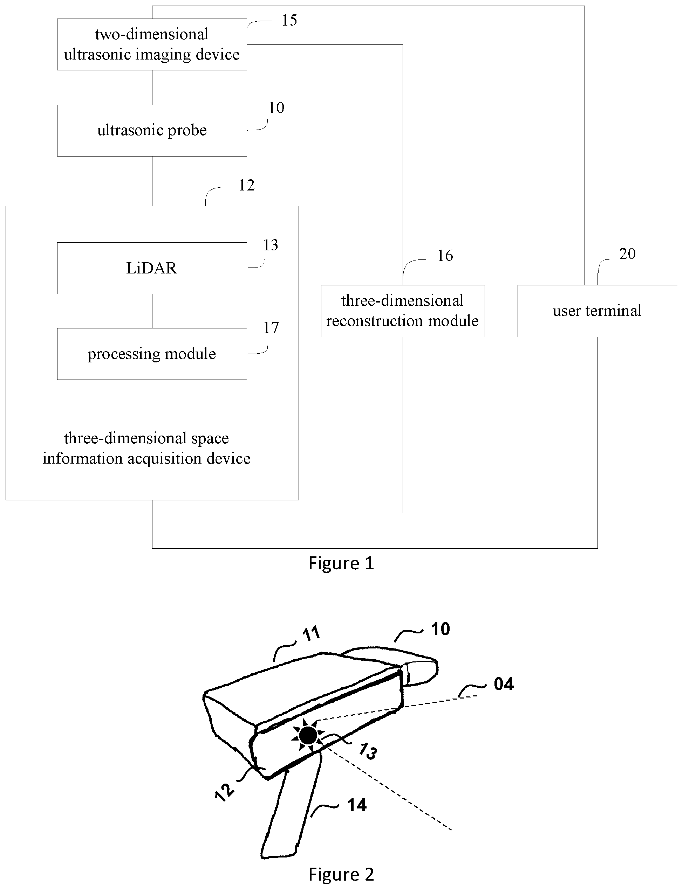

This embodiment provides a three-dimensional ultrasonic imaging system based on a LiDAR. Refer to Figure 1, which is a block diagram of the three-dimensional ultrasonic imaging system based on a LiDAR provided in this embodiment. As shown in Figure 1, the three-dimensional ultrasonic imaging system includes an ultrasonic probe 10, a two-dimensional ultrasonic imaging device 15, a three-dimensional space information acquisition device 12, a three-dimensional reconstruction module 16, and a user terminal 20. Wherein, the ultrasonic probe 10 is used to perform ultrasonic scanning on the region of interest of the target object; the two-dimensional ultrasonic imaging device 15 is communicatively connected to the ultrasonic probe 10, and its function is based on the ultrasonic scanning to generate a series of two-dimensional ultrasound images of the region of interest of the target object; the three-dimensional space information acquisition device 12 is communicatively connected to the ultrasonic probe 10, and is used to obtain the three-dimensional space information of the ultrasonic probe 10 through LiDAR; the three-dimensional reconstruction module 16 is communicatively connected to the two-dimensional ultrasonic imaging device 15 and the three-dimensional space information acquisition device 12, and is used to reconstruct a three-dimensional ultrasound image based on the three-dimensional space information of the ultrasonic probe 10 and the series of two-dimensional ultrasound images of the region of interest; the user terminal 20 is communicatively connected to the three-dimensional reconstruction module 16, and is used to display the three-dimensional ultrasound image.

In this embodiment, the two-dimensional ultrasonic imaging device 15 is built into the ultrasonic probe 10, forming an integrated unit with the ultrasonic probe 10, and is used to generate a series of two-dimensional ultrasound images of the region of interest of the target object. Here, the region of interest can be at least a portion of the target object or the entire object. The frequency, function, and appearance of the ultrasonic probe are not limited in the present application. The ultrasonic probe 10 and the two-dimensional ultrasonic imaging device 15 in this embodiment can be constructed by any ultrasonic probe and two-dimensional ultrasonic imaging device in the relevant field.

Of course, in some other embodiments, the two-dimensional ultrasonic imaging device 15 can be external to the ultrasonic probe 10 and connected to the ultrasonic probe 10 via a wired connection. In addition, the communication connection between the two-dimensional ultrasonic imaging device 15 and the ultrasonic probe 10 can also be a wireless connection.

In this embodiment, the three-dimensional space information acquisition device 12 includes a LiDAR 13 and a processing module 17 communicatively connected to the LiDAR 13. The function of the three-dimensional space information acquisition device 12 is to obtain the three-dimensional space information of the ultrasonic probe 10 in the three-dimensional space through the LiDAR 13. Here, the three-dimensional space information includes position and direction information.

In this embodiment, the three-dimensional reconstruction module 16 is communicatively connected to the two-dimensional ultrasonic imaging device 15 and the three-dimensional space information acquisition device 12 through a data integration and communication device (not shown in the diagram). The data integration and communication device integrate the series of two-dimensional ultrasound images obtained from the two-dimensional ultrasonic imaging device 15 and the series of three-dimensional space information obtained from the three-dimensional space information acquisition device 12, and transmits them to the three-dimensional reconstruction module 16 via wired or wireless mode. The three-dimensional reconstruction module 16 reconstructs the three-dimensional ultrasound image based on the three-dimensional space information of the ultrasonic probe 10 and the series of two-dimensional ultrasound images. It should be understood by those skilled in the art that any known reconstruction method in the field can be used to achieve the reconstruction of the three-dimensional ultrasound image, and therefore, it is not further described here.

In this embodiment, the user terminal 20 is equipped with an interactive application for the operator, primarily used for running control and displaying the three-dimensional ultrasound images. Those skilled in the art should be aware that the user terminal 20 can be a smartphone, smartwatch, desktop computer, laptop, tablet, smart TV, or similar devices.

The key aspect of the present application involves explaining how the three-dimensional space information acquisition device 12 is utilized to track and locate the moving ultrasound probe 10 within a three-dimensional space.

In this embodiment, a LiDAR 13 is mounted on the ultrasound probe 10. Furthermore, objects within the visible range of the LiDAR 13 are utilized as visual markers. Refer to Figure 2, which depicts a three-dimensional structure diagram of three-dimensional ultrasonic imaging system based on a LiDAR provided in this embodiment. The dashed line in Figure 2 represents the visual range of the LiDAR. As shown in Figure 2, the three-dimensional ultrasonic imaging system also includes an installation module 11 that connects the three-dimensional space information acquisition device 12 and the ultrasound probe 10. The bottom of the installation module 11 has a handle 14 that can be held by an operator to move the ultrasound probe 10 and conduct three-dimensional scans of the target object. The ultrasound probe 10 is connected to the LiDAR 13 via the installation module 11 and moves synchronously with it in the three-dimensional space. The ultrasound probe 10 is fixed on the front side of the installation module 11, while the LiDAR 13 is fixed on the right side of the installation module 11. The LiDAR 13 uses infrared laser ranging to observe the depth data of the surrounding environment and continuously compares it to obtain its own initial three-dimensional space information. The initial three-dimensional space information, after being processed by the processing module 17, can be used to obtain the three-dimensional space information of the ultrasound probe 10.

Specifically, during the ultrasonic scanning process of the ultrasound probe 10 on the region of interest of the target object, the three-dimensional space information acquisition device 12 obtains environmental depth data through the LiDAR 13 and generates initial three-dimensional space information based on the environmental depth data. The initial three-dimensional information refers to the LiDAR 13's own three-dimensional space information based on its self-capabilities, which is a function inherent to LiDAR sensors known to those skilled in the art. Therefore, it is not necessary to further elaborate on generating the LiDAR's own three-dimensional space information based on the environmental depth data. After obtaining the three-dimensional space information of the LiDAR 13, the processing module 17 performs spatial transformation on the initial three-dimensional space information to generate the actual three-dimensional space information reflecting the ultrasound probe 10 (including real-time position and direction information), and transmits the transformed three-dimensional space information of the ultrasound probe 10 to the three-dimensional reconstruction module 16 in a wired or wireless form. Specifically, when the ultrasound probe 10 moves, the three-dimensional space information acquisition device 12 converts the environmental depth data into a series of three-dimensional space information of the ultrasound probe 10 in real-time through the processing module 17. Preferably, the processing module 17 converts the environmental depth data into a series of three-dimensional space information of the ultrasound probe 10 in real-time using simultaneous localization and mapping (SLAM) technology. In a specific implementation, the ultrasound probe 10 starts moving from an unknown starting position in an unknown environment. During the movement of the ultrasound probe 10, laser rangefinders are used to repeatedly observe the environment features to obtain the environmental depth data for positioning its own position and pose, and an incremental map of the surrounding environment is constructed based on its own position, so as to achieve the purpose of obtaining the three-dimensional space information of the ultrasound probe 10. The advantage of this method is that large visual markers are used and placed at different positions in space. No matter how the position and angle are changed, the LiDAR 13 mounted on the ultrasound probe 10 can always detect certain markers.

Furthermore, to enhance the sensitivity of laser ranging and the effectiveness of three-dimensional tracking, the markers can be coated with infrared reflective material or equipped with LED lights or infrared lights that have self-illumination characteristics. It is worth noting that this application does not restrict the type, quantity, shape, combination, placement, or adhesive material of the markers.

Professionals in this field should be aware that the three-dimensional space information of the LiDAR 13 can be converted into the three-dimensional space information of the ultrasound probe 10 through known spatial transformation methods in this field. The details of this conversion are not reiterated in this application. The integration of the LiDAR 13 with the processing module 17 makes the three-dimensional ultrasonic imaging system of this application more compact and user-friendly. Furthermore, after obtaining the initial three-dimensional space information from the LiDAR 13, the processing module 17 performs preprocessing on it, including smoothing and/or noise reduction.

Professionals in this field should be aware that any device with a LiDAR sensor can be used to implement this embodiment, including but not limited to Velodyne's VLP-16 and similar devices that may be developed in the future.

It should be noted that this application does not restrict the placement of the LiDAR. In some other embodiments, the LiDAR 13 can be installed at any position on the installation module 11 or the ultrasound probe 10.

In summary, the embodiment described provides a three-dimensional ultrasonic imaging system based on LiDAR that has strong anti-interference capability, low cost, small size, and high accessibility. Furthermore, it can be used in any environment without the need for specific reference objects.

### The second embodiment

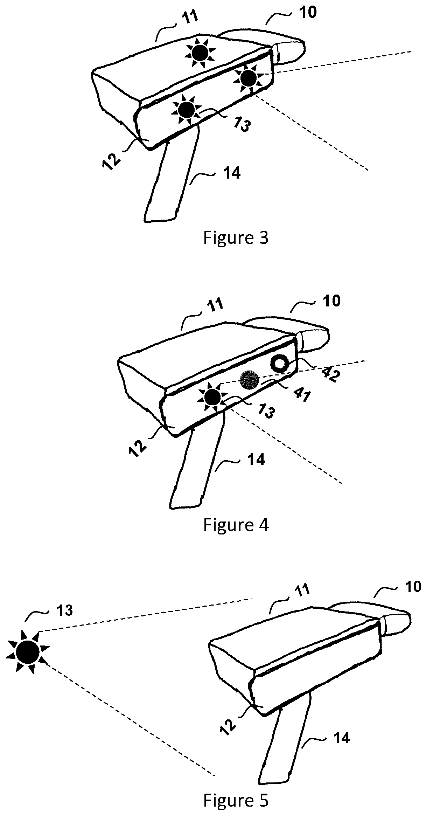

The embodiment provides a three-dimensional ultrasonic imaging system based on a LiDAR. The difference between this embodiment and the first embodiment is that there are multiple LiDARs 13 in the three-dimensional space information acquisition device 12. Refer to Figure 3, which is a schematic diagram of the three-dimensional structure diagram of the three-dimensional ultrasonic imaging system based on a LiDAR provided in this embodiment. The dashed line in the figure indicates the visual range of the LiDAR. As shown in Figure 3, the three-dimensional space information acquisition device 12 includes multiple LiDARs 13. In Figure 3, it can be seen that there are three LiDARs 13. Moreover, the three LiDARs 13 are installed at different positions on the installation module 11, which is connected to a single ultrasonic probe 10. The three LiDARs 13 are facing different directions. Therefore, by using the three LiDARs 13 to monitor the surrounding environment, multiple sets of initial three-dimensional space information can be obtained. The processing module 17 can generate the three-dimensional space information of the ultrasonic probe 10 based on multiple sets of initial three-dimensional space information in order to improve the accuracy of the generated three-dimensional space information. Those skilled in the art should be aware that there are various methods for processing multiple sets of initial three-dimensional space information, including simple averaging algorithms or other known methods. The present application is not limited to any specific method.

It is worth noting that the limitation of having a single LiDAR 13 is that the field of view (FOV) of the infrared laser is limited, and it cannot detect objects that are obstructed. Therefore, setting multiple LiDARs 13 allows when the ultrasonic probe 10 freely rotates in different directions infrared laser from more directions can be output, thereby enhancing the spatial resolution for the three-dimensional space.

It should be noted that the present application does not limit the number and placement of the LiDAR. In some other embodiments, the number of LiDARs can be any number (e.g., 2, 4, 5, 6, etc.), and the LiDARs can be arranged at any position on the installation module 11 or the ultrasonic probe 10.

### The third embodiment

This embodiment provides a three-dimensional ultrasonic imaging system based on a LiDAR. The difference between this embodiment and the first embodiment lies in that the three-dimensional space information acquisition device 12 further includes at least one motion sensor 41 and/or at least one camera 42. Referring to Figure 4, which is a three-dimensional structure diagram of the three-dimensional ultrasonic imaging system based on a LiDAR provided in this embodiment. The dashed line in the figure represents the visual range of the LiDAR. As shown in Figure 4, the three-dimensional space information acquisition device 12 includes a motion sensor 41 and a camera 42. The motion sensor 41 can be an accelerometer, a gyroscope, a magnetometer, or an inertial measurement unit (IMU). The motion sensor 41, which is mounted on the installation module 11 fixedly connected to the ultrasound probe 10, is used to obtain the acceleration and angular velocity values of the ultrasound probe 10, which can be used to calculate its movement distance and angle. The camera 42 mounted on the installation module 11 fixedly connected to the ultrasound probe 10 can obtain image information of the surrounding environment, and based on the changes in the obtained image information, calculate the position and direction of the ultrasound probe 10. Additionally, specific visual markers can be added to facilitate detection by the camera 42. The image information can be RGB and/or infrared intensity image information, depending on the type of camera used (such as a time-of-flight camera or a stereo camera for three-dimensional tracking). The application is not limited to any particular type of camera.

It is worth noting that the limitation of using a single LiDAR 13 is that it may be susceptible to external environmental interference. However, since the motion sensor 41 and camera 42 utilize different spatial sensing technologies, an occasional independent disturbance will not affect the overall performance of all the sensors (motion sensor 41 and camera 42). In this embodiment, motion sensors and/or cameras are used in conjunction with the LiDAR. The additional information from these sensors can be compared and combined with the ranging results from the LiDAR to reduce the interference caused by environmental factors and even correct the three-dimensional space information. Therefore, the purpose of combining multiple sensors with the LiDAR is to supplement the acquisition of three-dimensional space information and increase the accuracy of positioning.

The skilled person in this field should be aware that this embodiment can be implemented using LiDAR devices that integrate any existing cameras and/or motion sensors in the field, including but not limited to Intel's Realsense L515 and similar devices developed in the future. Taking Realsense L515 as an example, it comes with an integrated infrared laser emitter, an RGB camera, and an inertial measurement unit (IMU). The LiDAR camera is a depth camera that can provide multiple types of information simultaneously, including distance information between objects and the infrared laser emitter, a color RGB image, an infrared image reflecting infrared strength, and motion information such as angular velocity and acceleration.

### The fourth embodiment

This embodiment provides a three-dimensional ultrasonic imaging system based on a LiDAR. The difference between the three-dimensional ultrasonic imaging system provided in this embodiment and that provided in the first embodiment is that the LiDAR 13 is not set on the ultrasound probe 10. Instead, the LiDAR 10 is placed in a location where at least one marker is within the visible range of the LiDAR 10. Meanwhile, the ultrasound probe 10 itself or a part of the ultrasound probe 10 serves as the marker. See Figure 5, which is a three-dimensional structure diagram of the three-dimensional ultrasonic imaging system based on a LiDAR provided in this embodiment. The dashed line in the figure represents the visible range of the LiDAR. As shown in Figure 5, the LiDAR 10 and the ultrasound probe 10 are not physically connected or fixed together. The entire ultrasound probe 10 is within the visible range of the LiDAR 10. During the process of ultrasonic scanning for the area of interest in the target object, if the ultrasound probe 10 is moved, the three-dimensional space information acquisition device 12 obtains surrounding environmental depth data through the LiDAR 10 and continually compares it. In this embodiment, at least a part of the three-dimensional surface information (i.e. contour information) of the ultrasound probe 10 serves as the marker, which is detected and compared in real time by the LiDAR 10. In order to improve the sensitivity of laser ranging and the effectiveness of three-dimensional tracking, the marker can be coated with infrared reflective material or equipped with LED lights or infrared lights that have their own luminescent properties. Specifically, the infrared reflective coating is used to clearly distinguish the marker from the background under infrared illumination. It should be noted that the present application does not limit the type, number, shape, combination method, setting position, or attached material of the marker. The LiDAR 13 generates the initial three-dimensional space information of ultrasonic probe 10, which includes the position and orientation information of the marker, based on the variation and comparison results of the contour information of the marker. The initial three-dimensional space information is then transformed and processed by processing module 17 to generate three-dimensional space information that accurately reflects the motion of ultrasonic probe 10. The motion of ultrasonic probe 10 in three-dimensional space is being continuously monitored by the real-time surveillance of the LiDAR 13.

In some other embodiments, at least one specific visual marker can be placed at any position of the ultrasonic probe 10. This visual marker is also located within the visible range of the LiDAR 13.

Those skilled in the art should be aware that this embodiment can be implemented using any device with a LiDAR sensor in the field, including but not limited to Velodyne's VLP-16 and similar devices that may be developed in the future.

### The fifth embodiment

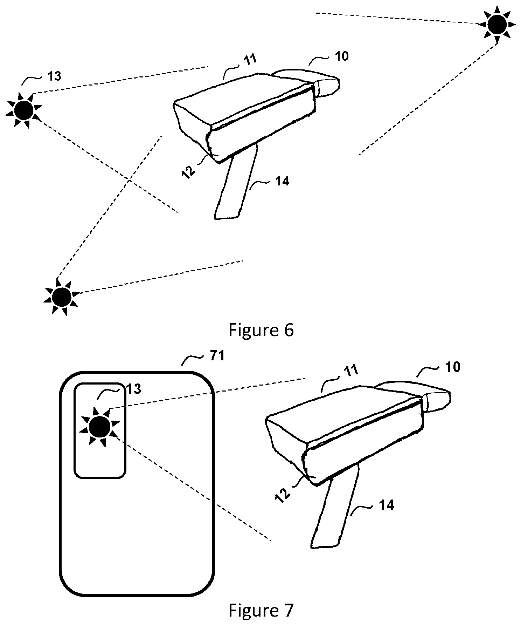

This embodiment provides a three-dimensional ultrasonic imaging system based on a LiDAR. The difference between this three-dimensional ultrasonic imaging system and that of the fourth embodiment is that there are multiple LiDARs in the three-dimensional space information acquisition device 12. Refer to Figure 6, which is a three-dimensional structure diagram of the three-dimensional ultrasonic imaging system based on a LiDAR provided by this embodiment. The dotted line in the figure indicates the visible range of the LiDAR. As shown in Figure 6, the three-dimensional space information acquisition device 12 includes multiple LiDARs 13. In Figure 6, it can be seen that there are three LiDARs 13. Furthermore, the three LiDARs 13 are positioned at different locations in space and are oriented in different directions. Each LiDAR 13 simultaneously records the position and orientation information of the ultrasonic probe 10. When the ultrasonic probe 10 rotates freely, at least one LiDAR 13 can promptly obtain the motion trajectory and pose of the ultrasonic probe 10. As a result, multiple sets of contour information of the ultrasonic probe 10 or a part thereof can be obtained through the three LiDARs. Based on these multiple sets of contour information, the initial three-dimensional space information of the ultrasonic probe 10 can be generated. The processing module 17 then transforms the multiple sets of initial three-dimensional space information to generate the three-dimensional space information of the ultrasonic probe 10. It is worth noting that a limitation of setting a single LiDAR 13 is that the field of view (FOV) of the infrared laser is limited, which cannot detect obstructed objects. Therefore, by setting multiple LiDARs 13 and combining multiple sets of ranging results, the stability and reliability of the system can be improved.

### The sixth embodiment

This embodiment provides a three-dimensional ultrasonic imaging system based on a LiDAR. The difference between this embodiment and the fourth embodiment is that the LiDAR is not integrated with the two-dimensional ultrasonic imaging device at the traditional sensor level. Instead, it organically integrates the existing terminal device integrated with a LiDAR with the two-dimensional ultrasound device to construct a three-dimensional ultrasonic imaging system at the device application level. The mentioned LiDAR 13 is integrated into the existing terminal device 71 to acquire a series of three-dimensional space information of the moving ultrasound probe 10. The terminal device 71 is positioned in a way that allows at least one marker to be within the visible range of the LiDAR 13, and the ultrasound probe 10 itself or a part of the ultrasound probe 10 serves as the marker. The three-dimensional space information acquisition device 12 is at least a portion of any terminal device 71 that is integrated with a LiDAR 13. Please refer to Figure 7. Figure 7 depicts a three-dimensional structure diagram of the three-dimensional ultrasonic imaging system based on a LiDAR provided in this embodiment, with the dashed line indicating the visible range of the LiDAR. As shown in Figure 7, the three-dimensional ultrasonic imaging system comprises a terminal device 71, on which the LiDAR 13 is installed. The terminal device 71 can be a smart phone, smart watch, tablet, or iPad. Preferably, it is the integration of two-dimensional ultrasonic imaging into an integrated smart terminal that already has a LiDAR and three-dimensional sensing function. It should be noted that the LiDAR 13 and corresponding processing module on the terminal device 71 are existing technologies, equipped with sensing capabilities and capable of obtaining depth data. Specifically, from the hardware perspective, the LiDAR 13 is one of the firmware components of the terminal device 71. From the software perspective, the terminal device 71 can use an application program to perform operational control of the three-dimensional ultrasonic imaging system, and execute the acquisition of two-dimensional images, post-processing of three-dimensional space information, reconstruction of three-dimensional images, and image display. Therefore, the terminal device 71, in combination with the ultrasound probe 10, enables the application of three-dimensional tracking. Building a three-dimensional ultrasonic imaging system using the terminal device 71 that is integrated with a LiDAR 13 has the following advantages: 1) promotes miniaturization, high integration, and portability of ultrasound devices; 2) enhances the accessibility of three-dimensional ultrasound technology; 3) expands the application scenarios in the field of three-dimensional sensing.

### The seventh embodiment

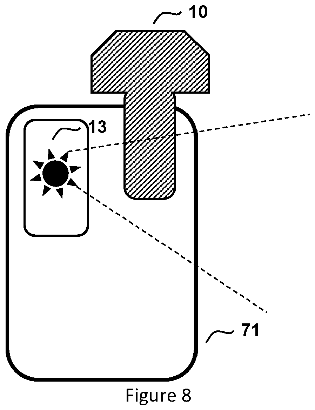

In this embodiment, a three-dimensional ultrasonic imaging system based on a LiDAR is provided. The difference between the three-dimensional ultrasonic imaging system provided in this embodiment and that in the sixth embodiment is that the ultrasound probe 10 is directly integrated with the terminal device 71, the LiDAR 13 on the terminal device 17 is synchronously moved with the ultrasound probe 10. Please refer to Figure 8, which is a three-dimensional structure diagram of the three-dimensional ultrasonic imaging system based on a LiDAR provided in this embodiment. The dotted line in the figure indicates the visible range of the LiDAR. As shown in Figure 8, the ultrasound probe 10 and the LiDAR 13 are configured on the same side of the terminal device 71. A portion of the ultrasound probe 10 is within the visual range of the LiDAR 13 and serves as a marker. When the operator handheld the device and moves it in space, it has the following functionalities: 1) ultrasonic scanning of the regions of interest on the target object is performed by the integrated ultrasound probe 10; 2) a series of three-dimensional space information of the moving ultrasound probe 10 is obtained by the integrated LiDAR 13, which uses a portion of the ultrasound probe 10 within its visual range as a marker; 3) the terminal device 71 is used to operate and control the system of the handheld device, enabling image acquisition, three-dimensional reconstruction, post-processing, and display.

Those skilled in this field should be aware that in the seventh embodiment and the sixth embodiment, any terminal device with an integrated LiDAR sensor in this field can be used, including but not limited to Apple's iPhone 12 Pro and iPad Pro, Samsung's Galaxy S20+ and S20 Ultra, and similar devices that may be developed in the future. Using iPhone 12 Pro as an example, its rear camera module comes with an integrated LiDAR scanner, a wide-angle camera, an ultra-wide-angle camera, and a telephoto camera. The camera module is a depth-sensing camera that provides distance information between markers and the camera, as well as environmental depth data, enabling functions such as augmented reality (AR), background blurring, object three-dimensional scanning and modeling.

### The eighth embodiment

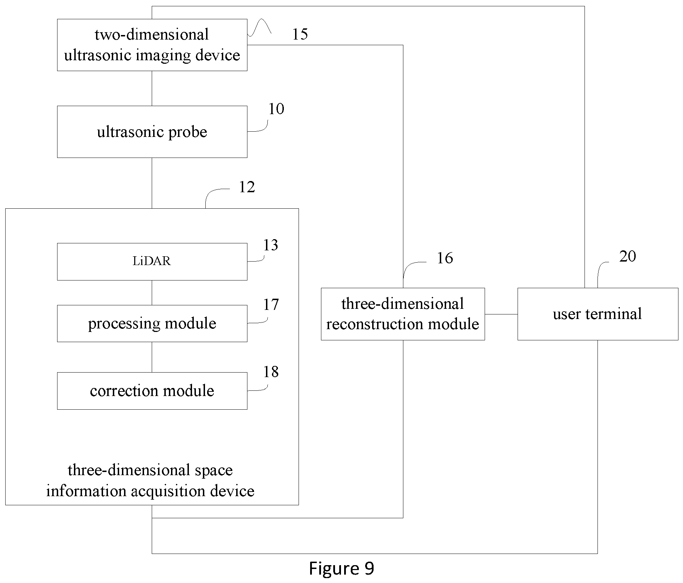

This embodiment provides a three-dimensional ultrasonic imaging system based on a LiDAR. Refer to Figure 9, which is a block diagram of the three-dimensional ultrasonic imaging system based on a LiDAR provided in this embodiment. As shown in Figure 9, the difference between this three-dimensional ultrasonic imaging system and that of the first embodiment is that the three-dimensional space information acquisition device 12 further includes a correction module 18. The correction module 18 is used to adjust the position of the initial three-dimensional space information and the two-dimensional ultrasound image in three-dimensional space based on the variations in the measured initial three-dimensional space information and the content of the series of two-dimensional ultrasound images.

Specifically, when the variation of the initial three-dimensional space information is greater than the three-dimensional space information variation threshold, and the variation of the content of the series of two-dimensional ultrasound images is smaller than the series of two-dimensional ultrasound images content variation threshold, the initial three-dimensional space information is corrected. The correction module 18 compares the variation in the initial three-dimensional space information and the variation in the content of the series of two-dimensional ultrasound images obtained during adjacent measurements or within a specific period of time. Based on the experience that the movement of the ultrasound probe 10 does not undergo sudden large changes in practical applications, the obtained series of two-dimensional ultrasound images will not suddenly change either. Therefore, if the variation in the content of the series of two-dimensional ultrasound images obtained by the two-dimensional ultrasonic imaging device 15 is very small, but the initial three-dimensional space information measured by the LiDAR 13 shows significant changes, it indicates that there is an error in the three-dimensional space information and it needs to be corrected. Under normal circumstances, if the ultrasound probe 10 suddenly moves while scanning the region of interest, the content of the series of two-dimensional ultrasound images will also change abruptly. Therefore, if there are significant changes in the measured three-dimensional space information, but the series of two-dimensional ultrasound images do not exhibit significant changes (based on the practical experience that the scanned target is usually stationary or moves very little and slowly), it can be inferred that there is an error in the measured three-dimensional space information and it needs to be corrected. The specific correction methods are as follows: 1) utilize the measured and determined correct three-dimensional space information, which consists of two or more points, to perform extrapolation on the three-dimensional space information values that need to be corrected; 2) after obtaining the next one or multiple correct three-dimensional space information values, interpolate them with the previous one or multiple correct three-dimensional space information values; 3) after the scanning is completed, use all the determined correct three-dimensional space information values to perform three-dimensional curve fitting in order to obtain the three-dimensional space information values that require correction.

When the variation of the initial three-dimensional space information is less than the three-dimensional space information variation threshold, and the variation of the content of the series of two-dimensional ultrasound images is greater than the series of two-dimensional ultrasound images content variation threshold, the correction module corrects the position of the series of two-dimensional ultrasound images in the three-dimensional space. Based on experience, if the scanned object moves, the content of the series of two-dimensional ultrasound images will show significant changes. Therefore, if the variation of the measured initial three-dimensional space information is very small, but there is a significant change in the content of the series of two-dimensional ultrasound images, it indicates that the region of interest of the target object has moved during the ultrasound scanning process. The correction module uses the initial three-dimensional space information to correct the position of the series of two-dimensional ultrasound images in the three-dimensional space. The specific correction methods are as follows: 1) utilize the measured and determined correct three-dimensional space information, which consists of two or more points, to perform extrapolation on the three-dimensional space information values that need to be corrected; 2) after obtaining the next one or multiple correct three-dimensional space information values, interpolate them with the previous one or multiple correct three-dimensional space information values; 3) after the scanning is completed, use all the determined correct three-dimensional space information values to perform three-dimensional curve fitting in order to obtain the three-dimensional space information values that require correction. Those skilled in the field can also use any other known correction methods in the field to achieve the correction of three-dimensional space information.

### The ninth embodiment

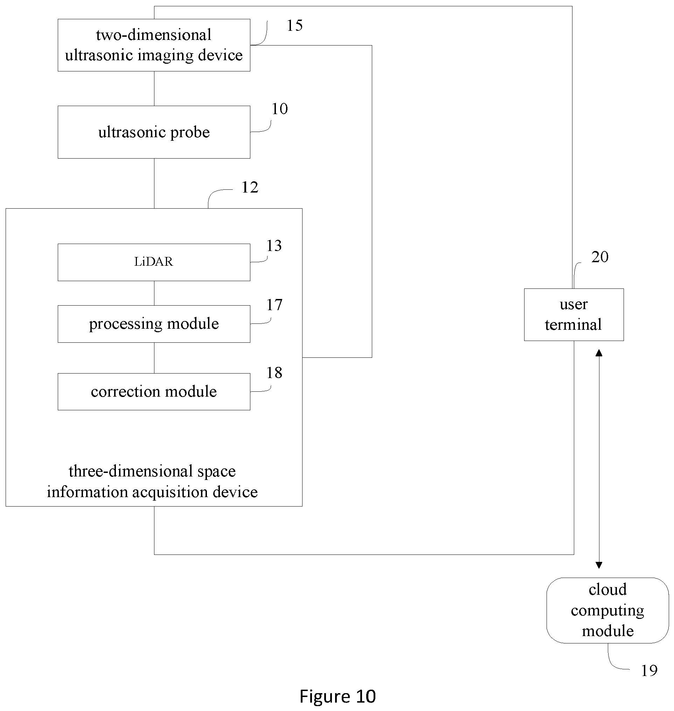

This embodiment provides a three-dimensional ultrasonic imaging system based on a LiDAR. Refer to Figure 10, which shows a block diagram of the three-dimensional ultrasonic imaging system based on a LiDAR in this embodiment. As shown in Figure 10, the difference between this embodiment and the first embodiment is that the three-dimensional ultrasonic imaging system includes a cloud computing module 19. The cloud computing module 19 replaces the three-dimensional reconstruction module and is responsible for implementing all the functions of the three-dimensional reconstruction module on a cloud server.

Those skilled in this field should be aware that the system connects to the cloud computing module 19 through user terminal 20 via wired or wireless transmission, enabling data exchange. Specifically, the system uploads the two-dimensional ultrasound images obtained from the two-dimensional ultrasonic imaging device 15 and the three-dimensional space information obtained from the three-dimensional space information acquisition device 12 to the cloud computing module 19 or similar processing units through the user terminal 20. The cloud computing module 19 performs analysis and processing on the data, executing cloud computing to reconstruct the three-dimensional ultrasound images. After analysis, computation, and processing in the cloud, the processed three-dimensional ultrasound images and related data are sent back to the user terminal 20 for display.

It is worth noting that the cloud computing module 19 stores various algorithms for image processing, three-dimensional reconstruction, and three-dimensional display. Specifically, it can be used to perform three-dimensional reconstruction on data that has not been processed by the local system. Additionally, it is preferable to set up a big data workstation on the cloud server for data storage, management, retrospective analysis, and sharing purposes.

By utilizing cloud servers to interact with the local system, the present application system can save local resources and computing power, and has the following advantages: 1) promotes hardware miniaturization and integration; 2) enhances system portability; 3) facilitates data management; 4) enables updates, optimizations, and execution of more advanced artificial intelligence algorithms.

### The tenth embodiment

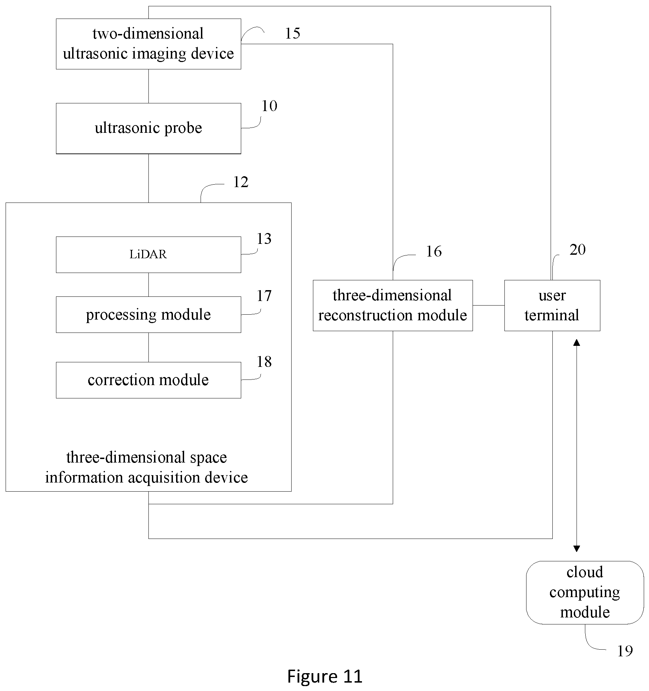

This embodiment provides a three-dimensional ultrasonic imaging system based on a LiDAR. Referring to FIG. 11, FIG. 11 is a block diagram illustrating the three-dimensional ultrasonic imaging system based on a LiDAR provided by this embodiment. As shown in FIG. 11, the difference between the three-dimensional ultrasonic imaging system provided in this embodiment and that of the first embodiment is that the system includes a cloud computing module 19, which is used to implement partial functions of the three-dimensional reconstruction module on a cloud server.

It should be known to those skilled in this field that the system connects to the cloud computing module 19 through a wired or wireless transmission method via the user terminal 20, enabling data exchange. Specifically, the system uploads the two-dimensional ultrasound images obtained by the two-dimensional ultrasonic imaging device 15 and the three-dimensional space information obtained by the three-dimensional space information acquisition device 12 to the cloud computing module 19 or similar processing units through the user terminal 20. The cloud computing module 19 analyzes and processes the data, performs cloud computing to reconstruct the three-dimensional ultrasound images. After analysis, computation, and processing in the cloud, the processed three-dimensional ultrasound images and relevant data are transmitted back to the user terminal 20 for display.

It is worth noting that the cloud computing module 19 stores various algorithms for image processing, three-dimensional reconstruction, and three-dimensional display. There are two scenarios: 1) it can further perform three-dimensional reconstruction on preprocessed data from the local system; 2) it can perform post-processing on data that has already undergone three-dimensional reconstruction in the local system. Additionally, it is preferable to set up a big data workstation on the cloud server for data storage, management, retrospective analysis, and sharing.

By utilizing cloud servers to interact with the local system, the present application system can save local resources and computing power, and has the following advantages: 1) promotes hardware miniaturization and integration; 2) enhances system portability; 3) facilitates data management; 4) enables updates, optimizations, and execution of more advanced artificial intelligence algorithms.

### The eleventh embodiment

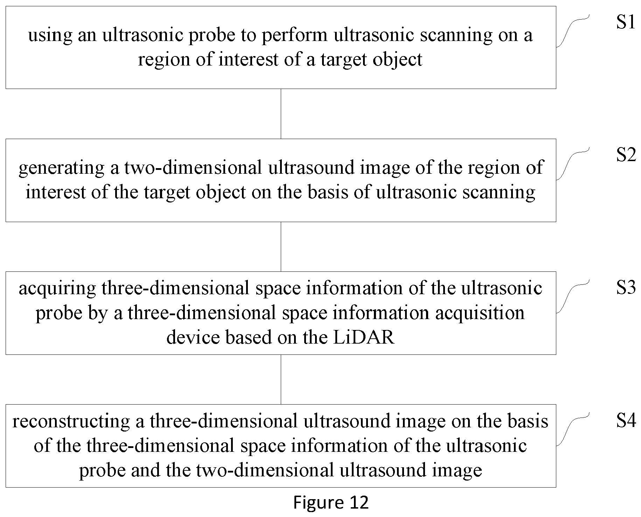

This embodiment provides a three-dimensional ultrasonic imaging method based on a LiDAR. Refer to Figure 12, which shows the process flow diagram of the three-dimensional ultrasonic imaging method based on a LiDAR provided by embodiment. As shown in Figure 12, the three-dimensional ultrasonic imaging method includes the following steps:
S 1. using an ultrasonic probe to perform ultrasonic scanning on a region of interest of a target object;
S2. generating a series of two-dimensional ultrasound images of the region of interest of the target object on the basis of ultrasonic scanning;
S3. acquiring three-dimensional space information of the ultrasonic probe by a three-dimensional space information acquisition device based on the LiDAR;
S4. reconstructing a three-dimensional ultrasound image on the basis of the three-dimensional space information of the ultrasonic probe and the series of two-dimensional ultrasound images.

Specifically, when the ultrasonic probe moves, the three-dimensional space information acquisition device converts the environmental depth data into a series of three-dimensional space information of the ultrasonic probe in real-time through the processing module. In a preferred embodiment, when the ultrasonic probe moves, the three-dimensional space information acquisition device uses the Simultaneous Localization and Mapping (SLAM) technology to convert the environmental depth data into a series of three-dimensional space information of the ultrasonic probe in real-time through the processing module.

In this embodiment, the LiDAR is installed on the ultrasonic probe. The three-dimensional space information acquisition device obtains its own three-dimensional space information and the three-dimensional space information of the ultrasonic probe that moves together with it. Therefore, the step S3 comprises: step S31. acquiring environmental depth data by the LiDAR of the three-dimensional space information acquisition device; step S32. generating an initial three-dimensional space information based on the environmental depth data; step S33. converting the initial three-dimensional space information into three-dimensional space information of the ultrasound probe.

In some other embodiments, the three-dimensional space information acquisition device 12 may include multiple LiDARs installed at different positions and facing different directions on a single ultrasonic probe 10 (see Figure 3 for example). Therefore, in these embodiments, step S3 includes: step S31, using multiple LiDARs installed at different positions or facing different directions on the ultrasonic probe to obtain multiple sets of environmental depth data; step S32, generating multiple sets of initial three-dimensional space information based on the multiple sets of environmental depth data; and step S33, transforming the multiple sets of initial three-dimensional space information to generate the three-dimensional space information of the ultrasonic probe. This method can improve the accuracy of spatially locating the ultrasonic probe.

In some other embodiments, the LiDAR is placed at a location where at least one marker is within the field of view of the LiDAR (see Figure 5 for example). The marker can be at least a part of the ultrasonic probe or at least a specific visual marker placed on the ultrasonic probe. The LiDAR is separated from the ultrasonic probe and does not move synchronously with the ultrasonic probe. Therefore, in these embodiments, step S3 comprises: step S31. acquiring a contour information of the marker by the LiDAR of the three-dimensional space information acquisition device; step S32. generating an initial three-dimensional space information of the ultrasound probe based on the contour information of the marker; step S33. converting the initial three-dimensional space information of the ultrasonic probe into the three-dimensional space information of the ultrasound probe.

In some other embodiments, the three-dimensional space information acquisition device 12 may include multiple LiDARs placed at different positions or facing different directions in space (see Figure 6 for example). The marker can be at least a part of the ultrasonic probe or at least a specific visual marker placed on the ultrasonic probe. The LiDAR is separated from the ultrasonic probe and does not move synchronously with the ultrasonic probe. Therefore, in these embodiments, step S3 includes: step S31: using multiple LiDARs positioned at different locations or facing different directions in space to acquire multiple sets of contour information of the marker; step S32: generating multiple sets of initial three-dimensional space information of the ultrasound probe based on the multiple sets of contour information of the marker; step S33: transforming the initial three-dimensional space information of the multiple sets of ultrasound probe to generate three-dimensional space information of the ultrasound probe. This method can improve the accuracy of spatial positioning of the ultrasound probe.

In some other embodiments, the following step is included between steps S32 and S33: based on the changes in the measured initial three-dimensional space information and the changes in the content of the series of two-dimensional ultrasound images, the positions of the initial three-dimensional space information and the series of two-dimensional ultrasound images in the three-dimensional space are corrected.

In some other embodiments, the three-dimensional ultrasonic imaging method of the present application can also utilize cloud servers for data analysis and processing, completing all or part of the three-dimensional image reconstruction work in the cloud. Therefore, in these embodiments, step S4 comprises: S41. transferring the three-dimensional space information of the ultrasonic probe and the series of two-dimensional ultrasound images to a cloud server; S42. based on the three-dimensional space information of the ultrasonic probe and the series of two-dimensional ultrasound images, reconstructing the three-dimensional ultrasound image at the cloud server; S43. transferring the three-dimensional ultrasound image to a user terminal for display.

In conclusion, the three-dimensional ultrasonic imaging method based on a LiDAR in this application obtain three-dimensional space information using LiDAR to reconstruct the three-dimensional ultrasound image in a flexible, low-cost and small-size manner, effectively avoiding interference, without the need for specific reference objects. Furthermore, this application can also integrate existing two-dimensional ultrasonic imaging device with intelligent terminals equipped with integrated LiDAR to build a three-dimensional ultrasonic imaging system at the device application level, promoting miniaturization and portability of ultrasound equipment, thereby improving accessibility of the three-dimensional ultrasonic technology, and expanding application scenarios in the field of three-dimensional sensing.

Further familiarization by those skilled in the field reveals that the three-dimensional ultrasonic imaging method and system based on LiDAR, as disclosed in this application, can complement and explain each other. The functionalities and steps recorded in each can be combined, integrated, or substituted with one another.

The above is further supplemented by describing certain important functions through functional modules, which are specifically defined herein for the convenience of description. When these important functions are appropriately implemented, it is permissible to modify the boundaries of these functional modules. Similarly, the boundaries and sequence of flowchart modules are specifically defined herein to illustrate certain important functions. For wider application, the boundaries and sequence of flowchart modules can be defined differently as long as these important functions can still be achieved. The changes in the boundaries and sequence of the aforementioned functional modules and flowchart modules should still be considered within the scope of protection of the claims.

The present application can also be implemented through a computer program product. The program contains all the features necessary to implement the method of the present application. When installed in a computer system, the program can implement the method of the present application. The computer program referred to in this document is any expression of a set of instructions that can be written in any programming language, code, or symbolic representation. This set of instructions enables a system to have information processing capabilities to directly implement specific functions or to achieve specific functionality after performing the following steps: a) translation into other languages, codes, or representations; b) representation in different formats.

Although the present application has been described through specific embodiments, those skilled in the art should understand that various changes and equivalent substitutions can be made to the present application without departing from its scope. In addition, for specific situations or materials, various modifications can be made to the present application without departing from the scope of the present application. Therefore, the present application is not limited to the disclosed specific embodiments, but should include all embodiments falling within the scope of the claims of the present application.

The foregoing description is for illustrative purposes only, and should not be construed as limiting the present application. Any modifications, equivalents, substitutions, and improvements made within the spirit and principles of the present application should be included within the scope of protection of the present application.

## Claims

1. A three-dimensional ultrasonic imaging system based on LiDAR, wherein, comprises:
an ultrasonic probe, used for performing ultrasonic scanning on a region of interest of a target object;
a two-dimensional ultrasonic imaging device, used for generating two-dimensional ultrasound images of the region of interest of the target object on the basis of ultrasonic scanning;
a three-dimensional space information acquisition device, used for acquiring three-dimensional space information of the ultrasonic probe by means of the LiDAR;
a three-dimensional reconstruction module, used for reconstructing a three-dimensional ultrasound image on the basis of the three-dimensional space information of the ultrasonic probe and the series of two-dimensional ultrasound images.

2. The three-dimensional ultrasonic imaging system based on a LiDAR according to claim 1, wherein, the three-dimensional space information acquisition device comprises: a LiDAR and a processing module; the LiDAR is fixedly connected to the ultrasonic probe, and moves synchronously with the ultrasonic probe; the LiDAR is used to acquire environmental depth data and generate an initial three-dimensional space information based on the environmental depth data; the processing module is used to convert the initial three-dimensional space information into three-dimensional space information of the ultrasound probe.

3. The three-dimensional ultrasonic imaging system based on a LiDAR according to claim 2, wherein, when the ultrasound probe is displaced, the three-dimensional space information acquisition device converts the environmental depth data in real-time through the processing module to obtain a series of three-dimensional space information of the ultrasound probe.

4. The three-dimensional ultrasonic imaging system based on a LiDAR according to claim 2, wherein, when the ultrasound probe is displaced, the three-dimensional space information acquisition device converts the environmental depth data in real-time through the processing module using simultaneous localization and mapping technology to obtain a series of three-dimensional space information of the ultrasound probe.

5. The three-dimensional ultrasonic imaging system based on a LiDAR according to claim 2, wherein, the three-dimensional space information acquisition device comprises multiple LiDARs; the relative positions of the multiple LiDARs with respect to the ultrasound probe are different, or the orientations of the multiple LiDARs are different; the multiple LiDARs are used to acquire multiple sets of environmental depth data and generate multiple sets of initial three-dimensional space information based on the multiple sets of environmental depth data; the processing module is used to transform the multiple sets of initial three-dimensional space information and generate the three-dimensional space information of the ultrasound probe.

6. The three-dimensional ultrasonic imaging system based on a LiDAR according to claim 1, wherein, the three-dimensional space information acquisition device comprises a LiDAR and a processing module; the LiDAR is separated from the ultrasound probe; the LiDAR is arranged in a position where at least one marker is within the visible range of the LiDAR, and is used to acquire a contour information of the marker and generate the initial three-dimensional space information of the ultrasound probe based on the contour information of the marker; the marker comprises at least a portion of the ultrasound probe and/or at least one visual marker set on the ultrasound probe; the processing module is used to convert the initial three-dimensional space information into three-dimensional space information of the ultrasound probe.

7. The three-dimensional ultrasonic imaging system based on a LiDAR according to claim 6, wherein, the three-dimensional space information acquisition device comprises multiple LiDARs; the multiple LiDARs are positioned at different locations or facing different directions in space, and are used to capture multiple sets of contour information of the ultrasound probe or a portion thereof; based on the multiple sets of the contour information of the ultrasound probe or a portion thereof, multiple sets of initial three-dimensional space information of the ultrasound probe are generated; the processing module is used for converting the multiple sets of initial three-dimensional space information into the three-dimensional space information of the ultrasound probe.

8. The three-dimensional ultrasonic imaging system based on a LiDAR according to claim 1, wherein, the three-dimensional space information acquisition device further comprises at least one motion sensor and/or at least one camera.

9. The three-dimensional ultrasonic imaging system based on a LiDAR according to claim 1, wherein, the three-dimensional space information acquisition device is at least a part of a terminal device integrated with the LiDAR.

10. The three-dimensional ultrasonic imaging system based on a LiDAR according to claim 1, wherein, the three-dimensional space information acquisition device also comprises a correction module; the correction module is used to correct the position of the initial three-dimensional space information and the two-dimensional ultrasound image in the three-dimensional space based on changes in the acquired initial three-dimensional space information and content of the series of two-dimensional ultrasound images.

11. The three-dimensional ultrasonic imaging system based on a LiDAR according to claim 1, wherein, the three-dimensional ultrasonic imaging system further comprises an installation module that securely connects the three-dimensional space information acquisition device and the ultrasound probe; the installation module comprises a handle that can be gripped by an operator.

12. The three-dimensional ultrasonic imaging system based on a LiDAR according to claim 1, wherein, the three-dimensional ultrasonic imaging system further comprises a data integration and communication device; the data integration and communication device is used to integrate the series of two-dimensional ultrasound images obtained from the two-dimensional ultrasonic imaging device and the three-dimensional space information obtained from the three-dimensional space information acquisition device, and transmit them to the three-dimensional reconstruction module through wired or wireless mode.

13. The three-dimensional ultrasonic imaging system based on a LiDAR according to claim 1, wherein, the three-dimensional ultrasonic imaging system further comprises a cloud computing module; the cloud computing module is used to implement all or part of functions of the three-dimensional reconstruction module.

14. The three-dimensional ultrasonic imaging system based on a LiDAR according to claim 1, wherein, the three-dimensional ultrasonic imaging system further comprises a user terminal; the user terminal is used to display the three-dimensional ultrasound image.

15. A three-dimensional ultrasonic imaging method based on a LiDAR, wherein, comprises the following steps:
step S 1, using an ultrasonic probe to perform ultrasonic scanning on a region of interest of a target object;
step S2, generating a series of two-dimensional ultrasound images of the region of interest of the target object on the basis of ultrasonic scanning;
step S3, acquiring three-dimensional space information of the ultrasonic probe by a three-dimensional space information acquisition device based on the LiDAR;
step S4, reconstructing a three-dimensional ultrasound image on the basis of the three-dimensional space information of the ultrasonic probe and the series of two-dimensional ultrasound images.

16. The three-dimensional ultrasonic imaging method based on a LiDAR according to claim 15, wherein, the LiDAR is fixedly connected to the ultrasonic probe, and moves synchronously with the ultrasonic probe, the step S3 comprises:
step S31, acquiring environmental depth data by the LiDAR of the three-dimensional space information acquisition device;
step S32, generating an initial three-dimensional space information based on the environmental depth data;
step S33, converting the initial three-dimensional space information into three-dimensional space information of the ultrasound probe.

17. The three-dimensional ultrasonic imaging method based on a LiDAR according to claim 15, wherein, the LiDAR is separated from the ultrasound probe; the LiDAR is arranged in a position where at least one marker is within the visible range of the LiDAR; the marker comprises at least a portion of the ultrasound probe and/or at least one visual marker set on the ultrasound probe; the step S3 comprises:
step S31, acquiring a contour information of the marker by the LiDAR of the three-dimensional space information acquisition device;
step S32, generating an initial three-dimensional space information of the ultrasound probe based on the contour information of the marker;
step S33, converting the initial three-dimensional space information of the ultrasonic probe into the three-dimensional space information of the ultrasound probe.

18. The three-dimensional ultrasonic imaging method based on a LiDAR according to claim 15, wherein, in the step S3, when the ultrasound probe is displaced, converting an environmental depth data in real-time to obtain a series of three-dimensional space information of the ultrasound probe.

19. The three-dimensional ultrasonic imaging method based on a LiDAR according to claim 15, wherein, in the step S3, when the ultrasound probe is displaced, converting an environmental depth data in real-time using simultaneous localization and mapping technology to obtain a series of three-dimensional space information of the ultrasound probe.

20. The three-dimensional ultrasonic imaging method based on a LiDAR according to claim 15, wherein, the step S4 comprises the following steps:
S41, transferring the three-dimensional space information of the ultrasonic probe and the two-dimensional ultrasound image to a cloud server;
S42, based on the three-dimensional space information of the ultrasonic probe and the series of two-dimensional ultrasound images, reconstructing the three-dimensional ultrasound image at the cloud server;
S43, transferring the three-dimensional ultrasound image to a user terminal for display.
